# FASCICULE DE BREVET EUROPEEN

(11) **EP 2 609 069 B1**
(45) Date de publication et mention de la délivrance du brevet: **24.09.2014**
(21) Numéro de dépôt: 11764853.5
(22) Date de dépôt: 17.08.2011
(51) Int. Cl.: C07C 67/08, C07C 69/44

(54) **PROCEDE DE PREPARATION D'ADIPATE DE DI-ALPHA-METHYLBENZYLE**
VERFAHREN FÜR DIE ZUBEREITUNG VON DI-ALPHA-METHYLBENZYLADIPAT
METHOD FOR PREPARING DI-ALPHA-METHYL BENZYL ADIPATE

(30) Priorité: 27.08.2010 FR 1056802
(43) Date de publication de la demande: 03.07.2013
(73) Titulaire: Rhodia Poliamida E Especialidades Ltda, Sao Paulo - SP (BR)
(72) Inventeur: RODRIGUES, Edson, 13632-460 Pirassununga (SP) (BR)
(74) Mandataire: Ridray, Annabelle
(86) Numéro de dépôt international: PCT/IB2011/001900
(87) Numéro de publication internationale: WO 2012/025810

(56) Documents cités:
- DE-A1- 19 943 436
- US-A- 2 755 262
- US-A- 3 818 071
- US-A- 4 007 218
- US-B1- 6 437 170
- SEEBACH D., HUNGERBÜHLER E., NAEF R., SCHNURRENBERGER P., WEIDMANN B., ZÜGER M.: "titanate mediated transesterification with functionalized substrates", SYNTHESIS, 1 janvier 1982 (1982-01-01), pages 138-141, XP002608305,

## Description

La présente invention concerne un procédé de préparation de l'adipate de di-alpha-méthylbenzyle par estérification directe de l'acide adipique et de l'alcool alpha-méthylbenzylique. Plus précisément, cette réaction procure des rendements élevés et est réalisée dans des conditions douces, en présence d'un catalyseur choisi parmi les orthotitanates de tétra-hydrocarbyle.

### CONTEXTE DE L'INVENTION

L'adipate de di-alpha-méthylbenzyle ou adipate de stirallyle est connu dans l'art comme plastifiant pour polymères (par exemple pour l'acétate de polyvinyle et le chlorure de polyvinyle, ou le polyéthylène chloré ou chlorosulfoné), mais il contribue également à améliorer d'autres fonctions, telles que la processabilité (par exemple pour les copolymères de styrène-acrylonitrile, les résines acrylonitrile butadiène-styrène, les résines méthacrylate de méthyle-butadiène-styrène), la fluidité (par exemple pour le moulage du polystyrène), les propriétés à basse température et de résistance à l'ozone (par exemple pour le vulcanisat du polyéthylène chloré ou chlorosulfoné).

L'art antérieur divulgue différentes manières de préparer l'adipate de dialpha-méthylbenzyle:
- US 2.755.262 - propose deux méthodes pour la préparation de l'ester di-alpha-méthylbenzyle: (i) par réaction de l'alcool alpha-méthylbenzylique avec le chlorure de l'acide dibasique, en présence d'une base tertiaire telle que la diméthylaniline, la pyridine, la quinoléine, en vue de la réaction avec le sous-produit chlorure d'hydrogène (ii) par l'échange d'ester entre l'alcool alpha-méthylbenzylique et le diméthyle ou de diéthyle de l'acide dibasique; en présence d'un alcoxyde métallique comme catalyseur.
- Zeinalov B. K. et al. (Synthesis of α-phenylethyl esters of dibasic aliphatic acids, and a study of their plasticizing properties. In Sin. Prevrashch. Monomernykh Soedin, 1967, pages 157-161, Bakou, URSS). Synthetized di-alpha methylbenzyl adipate by reaction of chloride alpha-methyl benzyl with adipic acid in the presence of triethylamine).
- JP 46038692 montre la préparation de l'adipate de di-alpha-méthylbenzyle par transestérification de l'alcool alpha-méthylbenzylique (alcool styrallylique - CAS 98-85-1) avec un adipate. La synthèse a utilisé l'adipate de diéthyle et un ratio molaire alcool alpha-méthylbenzylique: adipate de diéthyle = 2,05. Cette synthèse a été réalisée en présence de 0,6 parts d'ethoxide de sodium pour 37 parts d'éthanol, et utilise le toluène comme solvant (les réactifs ont été solubilisés dans 270 parts de toluène).
- JP 46038693 révèle que l'adipate de di-alpha-méthylbenzyle peut être préparé par réaction du chlorure de l'acide carboxylique avec l'alcool alpha-méthylbenzylique ou le chlorure d'alpha-méthyl benzyle avec l'acide carboxylique.

La recherche d'un procédé amélioré pour préparer l'adipate de di-alpha-méthylbenzyle est actuellement en cours, et vise à une production commercialement viable, avec des rendements améliorés dans des conditions douces - ce qui représente une réduction de la consommation d'énergie - afin de ne pas favoriser la formation de sous-produits indésirables qui requièrent des opérations de purification supplémentaires - ce qui représente une réduction du coût.

### DESCRIPTION DE L'INVENTION

La présente invention a pour principal objet un procédé de préparation d'adipate de di-alpha-méthylbenzyle par l'estérification de l'acide adipique et de l'alcool alpha-méthylbenzylique, en présence d'un catalyseur orthotitanate de tétra-hydrocarbyle . L'invention concerne notamment un procédé discontinu ou continu pour préparer l'adipate de di-alpha-méthylbenzyle par l'estérification catalytique directe, en présence d'orthotitanates de tétra-hydrocarbyle, de l'acide adipique et de l'alcool alphaméthylbenzilique.

Malgré les connaissances de base selon lesquelles les esters d'acides carboxyliques peuvent être préparés par réaction d'acides carboxyliques avec des alcools, l'encombrement stérique de l'alcool et de l'acide carboxylique a toujours été considéré comme un obstacle à ce type d'estérification. La Demandesse a été en mesure de surmonter les préjugés découlant de l'art antérieur et d'obtenir des rendements de conversion élevés grâce à une combinaison particulière des conditions d'estérification avec un choix judicieux des catalyseurs sélectifs tels que les orthotitanates de tétra hydrocarbyle, qui favorisent la réaction, et qui aident en même temps à éviter la formation de produits secondaires, par exemple, du styrène et de l'éther correspondant, au cours de l'estérification.

Un mode de réalisation particulier du procédé de l'invention comprend les étapes suivantes:
a. Dans un appareil doté d'une cuve de réaction avec une colonne à condensateur, et de moyens d'agitation, ont été chargés dans un ratio molaire entre l'alcool alpha-méthylbenzylique et l'acide adipique de 2,0:1 à 10:1, l'alcool alpha-méthylbenzylique correspondant un excès molaire de 10 à 20 % par rapport à l'acide adipique, et un catalyseur orthotitanate de tétra hydrocarbyle;
b. Le mélange a été chauffé jusqu'à environ 180°C.
c. L'azéotrope eau/alcool formé a été prélevé continuellement en particulier jusqu'à une acidité inférieure à 20 mg KOH/ g, plus particulièrement entre 10 et 5 mg KOH/g;
d. L'excès d'alcool alpha-méthylbenzylique a été prélevé;
e. L'acide adipique qui n'a pas réagi a été neutralisé et lavé.

Le procédé de l'invention peut éventuellement comporter ensuite les étapes indépendantes suivantes qui sont indépendantes entre elles:
- l'alcool résiduel alpha-methylbenzylique a été prélevé, notamment par stripping à la vapeur;
- le produit restant a été séché
- le produit restant a été filtré

Le procédé de l'invention est convenablement réalisé de manière continue ou discontinue, en particulier de manière discontinue.

Optionnellement, l'oxygène dissous est prélevé de l'alcool alpha-méthylbenzylique, avant son mélange avec l'acide et le catalyseur, et le chauffage du mélange dans le récipient réactionnel, et / ou l'adipate obtenu est soumis à un nettoyage en vue de la suppression de la couleur du produit réactionnel. Dans un mode particulier de réalisation, la totalité ou une partie de la réaction d'estérification est mise en oeuvre sous atmosphère inerte. Par exemple, un flux faible d'azote est employé pendant le chauffage des réactifs jusqu'à 120°C, jusqu'à ce que l'acide soit dissous dans l'alcool.

Dans un mode particulier de réalisation, le catalyseur est pré-dissous dans une quantité suffisante d'alcool, avant d'être ajouté au mélange d'alcool et d'acide. De manière particulière aussi, le mélange d'alcool alpha-méthylbenzylique et d'acide adipique est chauffé à la température réactionnelle, de préférence à environ 180°C, avant l'addition du catalyseur.

Les orthotitanates de tétra hydrocarbyle convenables répondent à la formule Ti(OR)₄, dans laquelle R est un radical hydrocarboné aliphatique cyclique, linéaire ou ramifié comportant de 1 à 24 atomes de carbone, en particulier de 1 à 6 atomes de carbone. Comme orthotitanates convenables ont peut citer, sans que cette liste soit limitative: les titanates de tétraméthyle, tétraéthyle, tétra-n-butyle, tétraisobutyle, tétra-sec-butyle, tétra-tert-butyl, tétraisopropyle, tétraoctadecyle, tétraphényle, tétra-n-pentyl-tétra-n-pentényle-et tétra-n-hexyle. Une quantité adéquate de ce catalyseur est comprise entre 10⁻⁶% et 1%, en particulier entre 0,001% et 0,003% du poids du mélange d'acide adipique et d'alcool alpha-méthylbenzylique.

En particulier, bien que cela ne soit pas obligatoire, le procédé d'estérification de l'invention n'utilise pas d'entraîneurs pour extraire l'eau formée à partir de la cuve de réaction afin de déplacer l'équilibre thermodynamique vers l'adipate. Au lieu de cela, la température utilisée dans la réaction favorise la formation d'un azéotrope binaire entre l'alcool alpha-méthylbenzylique (11%) et l'eau (89%), avec un point d'ébullition à 93,5°C, permettant que l'azéotrope soit extrait de la cuve de réaction pour éliminer l'eau formée. Les densités des phases formées après refroidissement sont très proches, donc difficiles à séparer, alors de préférence un excès molaire d'alcool d'environ 10% à environ 20% est utilisé par rapport aux exigences stoechiométriques, et l'alcool prélevé n'est pas recyclé dans le réacteur.

Un ratio molaire alcool alpha-méthylbenzylique/acide adipique qui convient à la réaction est de 2,0:1 à 10:1, notamment de 2,0: 1 à 10:2, en particulier de 2,4:1 à 2,8:1. La teneur d'alcool alpha-méthylbenzylique peut représenter un d'excès de 10 à 20 % molaire par rapport à la quantité stoechiométrique.

Également dans un mode particulier de réalisation, lorsque la réaction atteint une conversion à 70%, la quantité d'eau produite par la réaction diminue, donc la quantité d'azéotrope est également réduite. Ainsi, la pression est réduite, afin de maintenir la température du mélange réactionnel au-dessus du point d'ébullition. De préférence, la réduction de la pression est lente, jusqu'à 20 mm Hg.

L'élimination de l'excès d'alcool est réalisée en particulier à environ 180°C sous 20 mmHg, et l'alcool prélevé n'est plus recyclé dans le réacteur.

Les étapes utilisées pour la préparation de l'adipate de di-alpha-méthylbenzyle, après l'étape d'estérification, sont décrites en détail ci-dessous :
a. L'acide adipique qui n'a pas réagi est éliminé par neutralisation et lavage. Par exemple, une solution aqueuse de carbonate de sodium est ajoutée à la solution résultante à 50-55°C pour effectuer la neutralisation d'un acide qui n'a pas réagi et l'hydrolyse du catalyseur. L'utilisation d'une solution alcaline forte, par exemple d'hydroxyde de sodium ou de potassium, est moins préférée, car elle est plus susceptible de provoquer l'hydrolyse de l'adipate de di-α-méthylbenzyle, rendant plus difficile de neutraliser l'acidité à un niveau acceptable dans le cas des plastifiants. Après neutralisation de l'acidité résiduelle, la phase aqueuse est prélevée du mélange réactionnel et le produit de réaction est traité, par exemple par lavage avec une solution de chlorure de sodium à 10% en poids afin d'éliminer les monoesters résiduels.
b. L'alcool alpha-méthylbenzylique résiduel est éliminé par stripping à la vapeur ou de toute autre manière adéquate.

Certains alcools résiduels n'ayant pas réagi, environ 1-2% en poids, même après le prélèvement de l'excès d'alcool, restent le plus souvent dans la phase organique lors de la neutralisation et des étapes de lavage Si cela est souhaitable, un stripping à la vapeur à environ 6,5 kgf/cm2 peut être utilisé pour éliminer l'alcool et une partie de l'éther indésirable qui s'est formé.
c. Le jaunissement de l'ester peut être évité ou supprimé par une étape de nettoyage.

Une couleur jaune, parfois présente sur l'adipate formé est probablement liée à la formation d'impuretés dont la coloration est promue ou accélérée par l'incorporation d'oxygène au cours de la réaction d'estérification. Cela peut être considérablement réduit en éliminant préalablement l'oxygène dissous dans l'alcool de départ, par exemple par barbotage de gaz inerte exempt d'oxygène (par exemple de l'azote) dans l'alcool avant d'ajouter l'acide, le catalyseur et de les chauffer. Ou alors, le léger jaunissement du produit final, qui a été acquis au cours du procédé, peut également être éliminé, par exemple en passant le produit final à travers un filtre contenant du charbon actif, ou en mettant le produit final en contact avec du charbon actif, et réalisant par la suite une filtration. L'utilisation du peroxyde d'hydrogène est moins préférée, car bien qu'elle puisse diminuer le jaunissement, elle peut par contre augmenter l'acidité.

La température d'estérification adéquate est d'environ 180°C. Les températures proches de 190°C et au-dessus favorisent la décomposition thermique de l'adipate de di-alpha-méthylbenzyle et la déshydratation et la condensation de l'alcool alpha-méthylbenzylique.

Le suivi de l'étape d'estérification elle-même est effectué notamment par titration du mélange réactionnel afin de déterminer son acidité et/ou par mesure de l'eau produite à partir du mélange réactionnel.

Le séchage optionnel du produit final est réalisé en particulier sous faible pression, par exemple 70 mmHg, à une température d'environ 70°C, jusqu'à ce qu'une humidité cible désirée soit atteinte, par exemple de 0,2%, mesurée par exemple avec un appareil Karl Fischer.

### EXEMPLE

Dans un réacteur approprié doté de moyens d'agitation et d'un condenseur de tête, on a mis en oeuvre le mode de réalisation suivant de la présente invention :
- 445 kg d'acide adipique et 749 kg d'alcool styrallylique ont été chargés dans le réacteur, à la température ambiante.
- la température du mélange a été portée à 20° C, en présence d'un flux faible d'azote, sous agitation, jusqu'à ce que l'acide soit dissous dans l'alcool.
- la température a été alors portée à 180°C, sous pression ambiante, et 0,0267 kg de titanate de tétra-butyle (Tyzor^{™} TNTB, commercialisé par la société Dupont de Nemours), dissous dans l'alcool, ont été ajoutés.
- sous pression ambiante, l'azéotrope eau / alcool a été continuellement prélevé à partir de la tête du condenseur pendant que la réaction a été poursuivie jusqu'à une conversion de 70%, puis la pression a été réduite lentement et maintenue à environ 20 mmHg, jusqu'à ce que le mélange réactionnel atteigne un taux d'acidité de moins de 20 mg KOH / g, plus particulièrement entre 10 et 5 mg KOH/g.
- la température a été réduite à environ 55°C, et 19,6 kg de carbonate de sodium en solution aqueuse ont été ajoutés, et mélangés sous agitation pendant 30 minutes, afin de neutraliser le produit final. Ensuite, le produit réactionnel a été lavé avec 13,1 kg de NaCl (solution à 10% en poids). Le mélange a été laissé au repos, puis les phases séparées et la phase inférieure a été prélevée.
- le produit restant a été alors soumis à un stripping à la vapeur, à 6,5 kgf/cm2 à 140°C sous une pression de 30 mmHg, pendant 3 heures.
- Finalement, le produit final a été séché à une pression de 40 mmHg, à 70°C, jusqu'à ce que l'humidité soit d'environ de 0,2% en poids.

Le rendement de ce procédé a été de 92% sur une durée de 40h, et le taux de conversion a été de 96% sur 24 h.

## Revendications

1. PROCÉDÉ de préparation d'adipate de di-alpha-méthylbenzyle par l'estérification de l'acide adipique et de l'alcool alpha-méthylbenzylique, en présence d'un catalyseur orthotitanate de tétra-hydrocarbyle .

2. PROCÉDÉ selon la revendication 1, **caractérisé en ce que** ledit orthotitanate de tétra-hydrocarbyle est choisi parmi un ou plusieurs titanates de tétraméthyle, tétraéthyle, tétra-n-butyle, tétraisobutyle, tétra-sec-butyle, tétra-tert-butyle, tétraisopropyle, tétraoctadecyle, tétraphényle, tétra-n-pentyl-tétra-n-pentényle et tétra-n-hexyle.

3. PROCÉDÉ selon la revendication 1 **caractérisé en ce que** la teneur dudit othotitanate de tétra-hydrocarbyle varie de 10⁻⁶% à 1%, en particulier de 0,001% à 0,003% en poids du poids du mélange réactif d'acide adipique et d'alcool alpha-méthylbenzylique.

4. PROCÉDÉ selon la revendication 1, **caractérisé en ce que** le rapport molaire alcool alpha-méthylbenzylique: l'acide adipique varie de 2,0:1 à 10:1, notamment de 2.4:1 à 2,8:1.

5. PROCÉDÉ selon la revendication 1, **caractérisé en ce que** la teneur d'alcool alpha-méthylbenzylique représente un d'excès de 10 à 20% molaire par rapport à la quantité stoechiométrique.

6. PROCÉDÉ selon la revendication 1, **caractérisé en ce que**, dans un appareil comprenant une cuve de réaction dotée d'une colonne à condensateur, et de moyens d'agitation, il comprend les étapes suivantes:
a. charger l'alcool alpha-méthylbenzylique et l'acide adipique dans un rapport molaire de 2,0: 1 à 10:1, l'alcool alpha-méthylbenzylique correspondant à un excès molaire de 10 à 20%, et un catalyseur orthotitane d' hydrocarbyle;
b. Chauffer le mélange jusqu'à environ 180°C;
c. Prélever continuellement l'azéotrope eau/alcool formé, en particulier jusqu'à une acidité inférieure à 20 mg/g KOH, plus particulièrement entre 10 et 5 mg KOH/g;
d. Prélever l'excès d'alcool alpha-méthylbenzylique;
e. Neutraliser et laver l'acide adipique qui n'a pas réagi;

7. PROCÉDÉ selon la revendication 6, **caractérisé en ce qu'**il qu'il comprend les étapes suivantes qui sont indépendantes entre elles :
- Eliminer l'alcool résiduel alpha-methylbenzylique, notamment par stripping à la vapeur;
- Sécher le produit restant
- Filtrer le produit restant

8. PROCÉDÉ selon la revendication 6, **caractérisé en ce que** l'oxygène dissous est prélevé de l'alcool alpha-méthylbenzylique avant la réaction, en particulier par barbotage d'un gaz inerte exempt d'oxygène.

9. PROCÉDÉ selon la revendication 6, **caractérisé en ce que**, dans l'étape c, lorsque la conversion a atteint 70%, la pression est réduite, en particulier à 20 mmHg.

10. PROCÉDÉ selon la revendication 6, **caractérisé en ce que** la totalité ou partie de la réaction d'estérification est réalisée sous atmosphère inerte, telle que de l'azote.

11. PROCÉDÉ selon la revendication 6, **caractérisé en ce que** le catalyseur est pré-dissous dans une quantité suffisante d'alcool alpha-méthylbenzylique avant d'être ajouté au mélange d'alcool et d'acide.

12. PROCÉDÉ selon la revendication 6, **caractérisé en ce que** le mélange d'acide et d'alcool est chauffé, notamment jusqu'à 120°C, jusqu'à ce que l'acide soit entièrement dissous dans l'alcool alpha-méthylbenzylique, avant que le catalyseur soit ajouté au mélange.

13. PROCÉDÉ selon la revendication 6, **caractérisé en ce que** le mélange d'alcool alpha-méthylbenzylique et d'acide adipique est chauffé à la température de réaction, avant que le catalyseur est ajouté.

14. PROCÉDÉ selon la revendication 6, **caractérisé en ce que** l'élimination de l'excès d'alcool alpha-méthylbenzylique à l'étape d est effectué sous basse pression, telle que 20 mmHg.

## Patentansprüche

1. VERFAHREN zur Herstellung von Adipinsäuredi-alphamethylbenzylester durch Veresterung von Adipinsäure mit alpha-Methylbenzylalkohol in Gegenwart eines Tetrahydrocarbylorthotitanat-Katalysators.

2. VERFAHREN nach Anspruch 1, **dadurch gekennzeichnet, dass** das Tetrahydrocarbylorthotitanat aus einem oder mehreren Tetramethyl-, Tetraethyl-, Tetra-n-butyl-, Tetraisobutyl-, Tetra-sec-butyl-, Tetra-tert-butyl-, Tetraisopropyl-, Tetraoctadecyl-, Tetraphenyl-, Tetra-n-pentyl-, Tetra-n-pentenyl- und Tetra-n-hexyltitanaten ausgewählt wird.

3. VERFAHREN nach Anspruch 1, **dadurch gekennzeichnet, dass** der Gehalt des Tetrahydrocarbylorthotitanats von 10⁻⁶ bis 1 Gew.-%, insbesondere von 0,001 bis 0,003 Ges.-%, bezogen auf das Gewicht der reaktiven Mischung von Adipinsäure und alpha-Methylbenzylalkohol, variiert.

4. VERFAHREN nach Anspruch 1, **dadurch gekennzeichnet, dass** das Molverhältnis von alpha-Methylbenzylalkohol zu Adipinsäure von 2,0:1 bis 10:1, insbesondere von 2,4:1 bis 2,8:1, variiert.

5. VERFAHREN nach Anspruch 1, **dadurch gekennzeichnet, dass** der Gehalt an alpha-Methylbenzylalkohol einen Überschuss von 10 bis 20 Mol-% gegenüber der stöchiometrischen Menge darstellt.

6. VERFAHREN nach Anspruch 1, **dadurch gekennzeichnet, dass** es in einer Apparatur mit einem Reaktionsgefäß, das mit einer Kondensationskolonne und Rühreinrichtungen versehen ist, die folgenden Schritte umfasst:
a. Eintragen des alpha-Methylbenzylalkohols und der Adipinsäure in einem Molverhältnis von 2,0: 1 bis 10:1, wobei der alpha-Methylbenzylalkohol einem Überschuss von 10 bis 20 Mol-% entspricht, und eines Hydrocarbylorthotitanat-Katalysators;
b. Erhitzen der Mischung auf ungefähr 180°C;
c. kontinuierliches Austragen des gebildeten Wasser/Alkohol-Azeotrops, insbesondere bis zu einer Acidität von weniger als 20 mg/g KOH, spezieller zwischen 10 und 5 mg KOH/g;
d. Austragen des Überschusses von alpha-Methylbenzylalkohol;
e. Neutralisieren und Auswaschen der nicht abreagierten Adipinsäure.

7. VERFAHREN nach Anspruch 6, **dadurch gekennzeichnet, dass** es die folgenden, voneinander unabhängigen Schritte umfasst:
- Entfernen von restlichem alpha-Methylbenzyl-alkohol, insbesondere durch Dampfstrippen;
- Trocknen des verbleibenden Produkts;
- Filtrieren des verbleibenden Produkts.

8. VERFAHREN nach Anspruch 6, **dadurch gekennzeichnet, dass** der gelöste Sauerstoff vor der Reaktion aus dem alpha-Methylbenzylalkohol ausgetragen wird, insbesondere durch Spülen mit einem sauerstofffreien Inertgas.

9. VERFAHREN nach Anspruch 6, **dadurch gekennzeichnet, dass** in Schritt c beim Erreichen eines Umsatzes von 70% der Druck verringert wird, insbesondere auf 20 mmHg.

10. VERFAHREN nach Anspruch 6, **dadurch gekennzeichnet, dass** die Veresterungsreaktion ganz oder teilweise unter Inertatmosphäre, wie Stickstoff, durchgeführt wird.

11. VERFAHREN nach Anspruch 6, **dadurch gekennzeichnet, dass** der Katalysator vor der Zugabe zu der Mischung von Alkohol und Säure in einer ausreichenden Menge alpha-Methylbenzylalkohol vorgelöst wird.

12. VERFAHREN nach Anspruch 6, **dadurch gekennzeichnet, dass** die Mischung von Säure und Alkohol vor der Zugabe des Katalysators zu der Mischung erhitzt wird, insbesondere auf 120°C, bis die Säure vollständig in dem alpha-Methylbenzylalkohol gelöst ist.

13. VERFAHREN nach Anspruch 6, **dadurch gekennzeichnet, dass** die Mischung von alpha-Methylbenzylalkohol und Adipinsäure vor der Zugabe des Katalysators auf die Reaktionstemperatur erhitzt wird.

14. VERFAHREN nach Anspruch 6, **dadurch gekennzeichnet, dass** die Entfernung des Überschusses von alpha-Methylbenzylalkohol in Schritt d unter niedrigem Druck, wie 20 mmHg, durchgeführt wird.

## Claims

1. Process for the preparation of di(α-methylbenzyl) adipate by the esterification of adipic acid and α-methylbenzyl alcohol in the presence of a tetrahydrocarbyl orthotitanate catalyst.

2. Process according to Claim 1, **characterized in that** said tetrahydrocarbyl orthotitanate is chosen from one or more tetramethyl, tetraethyl, tetra(n-butyl), tetraisobutyl, tetra(sec-butyl), tetra(tert-butyl), tetraisopropyl, tetraoctadecyl, tetraphenyl, tetra(n-pentyl), tetra(n-pentenyl) and tetra(n-hexyl) titanates.

3. Process according to Claim 1, **characterized in that** the content of said tetrahydrocarbyl orthotitanate varies from 10⁻⁶% to 1%, in particular from 0.001% to 0.003%, by weight of the weight of the reactive mixture of adipic acid and α-methylbenzyl alcohol.

4. Process according to Claim 1, **characterized in that** the α-methylbenzyl alcohol/adipic acid molar ratio varies from 2.0:1 to 10:1, in particular from 2.4:1 to 2.8:1.

5. Process according to Claim 1, **characterized in that** the α-methylbenzyl alcohol content represents an excess of 10 to 20 mol%, with respect to the stoichiometric amount.

6. Process according to Claim 1, **characterized in that**, in an apparatus comprising a reaction vessel having a condensation column and having stirring means, it comprises the following stages:
a. charging the α-methylbenzyl alcohol and the adipic acid in a molar ratio of 2.0:1 to 10:1, the α-methylbenzyl alcohol corresponding to a molar excess of 10% to 20%, and a hydrocarbyl orthotitanate catalyst;
b. heating the mixture up to approximately 180°C;
c. continuously withdrawing the water/alcohol azeotrope formed, in particular up to an acidity of less than 20 mg/g KOH, more particularly between 10 and 5 mg KOH/g;
d. withdrawing the excess α-methylbenzyl alcohol;
e. neutralizing and washing out the unreacted adipic acid.

7. Process according to Claim 6, **characterized in that** it comprises the following stages, which are independent of one another:
- removing the residual α-methylbenzyl alcohol, in particular by steam stripping;
- drying the remaining product;
- filtering the remaining product.

8. Process according to Claim 6, **characterized in that** the dissolved oxygen is withdrawn from the α-methylbenzyl alcohol before the reaction, in particular by sparging with an oxygen-free inert gas.

9. Process according to Claim 6, **characterized in that**, in stage c, when the conversion has reached 70%, the pressure is reduced, in particular to 20 mmHg.

10. Process according to Claim 6, **characterized in that** all or a portion of the esterification reaction is carried out under an inert atmosphere, such as nitrogen.

11. Process according to Claim 6, **characterized in that** the catalyst is predissolved in a sufficient amount of α-methylbenzyl alcohol, before being added to the mixture of alcohol and acid.

12. Process according to Claim 6, **characterized in that** the mixture of acid and alcohol is heated, in particular up to 120°C, until the acid is completely dissolved in the α-methylbenzyl alcohol, before the catalyst is added to the mixture.

13. Process according to Claim 6, **characterized in that** the mixture of α-methylbenzyl alcohol and adipic acid is heated to the reaction temperature, before the catalyst is added.

14. Process according to Claim 6, **characterized in that** the removal of the excess α-methylbenzyl alcohol in stage d is carried out under low pressure, such as 20 mmHg.
